# EUROPEAN PATENT SPECIFICATION

(11) **EP 2 500 036 B1**
(45) Date of publication and mention of the grant of the patent: **07.05.2014**
(21) Application number: 11158861.2
(22) Date of filing: 18.03.2011
(51) Int. Cl.: A61K 39/395, A61P 35/00

(54) **MET inhibitors for enhancing radiotherapy efficacy**
MET-Hemmer zur Verbesserung der Wirksamkeit von Röntgentherapie
Inhibiteurs MET pour améliorer l'efficacité de radiothérapie

(43) Date of publication of application: 19.09.2012
(73) Proprietor: Metheresis Translational Research SA, 6900 Lugano (CH)
(72) Inventor: Comoglio, Paolo Maria, 10133 Torino (IT); Boccaccio, Carla, 10133 Torino (IT); Petronzelli, Fiorella, 00128 Roma (IT); De Santis, Rita, 00040 Pomezia (Roma) (IT)
(74) Representative: Freyria Fava, Cristina

(56) References cited:
- WO-A1-2005/016382
- WO-A1-2007/090807
- LAL BACHCHU ET AL: "Targeting the c-Met pathway potentiates glioblastoma responses to gamma-radiation", CLINICAL CANCER RESEARCH, THE AMERICAN ASSOCIATION FOR CANCER RESEARCH, US, vol. 11, no. 12, 15 June 2005 (2005-06-15) , pages 4479-4486, XP002551246, ISSN: 1078-0432, DOI: DOI:10.1158/1078-0432.CCR-05-0166
- AEBERSOLD D M ET AL: "Involvement of the hepatocyte growth factor/scatter factor receptor c-met and of Bcl-xL in the resistance of oropharyngeal cancer to ionizing radiation", INTERNATIONAL JOURNAL OF CANCER, JOHN WILEY & SONS, INC, UNITED STATES, SWITZERLAND, GERMANY, vol. 96, no. 1, 20 February 2001 (2001-02-20), pages 41-54, XP002551247, ISSN: 0020-7136, DOI: DOI:_ [retrieved on 2001-02-05]

## Description

### FIELD OF THE INVENTION

The present disclosure concerns the use of MET inhibitors for enhancing the efficacy of radiotherapy in patients suffering from cancers.

### TECHNICAL BACKGROUND

Although successfully employed to treat cancer patients, radiotherapy can fail to eradicate the tumour, which relapses with a more aggressive phenotype. Consistently, a paradoxical pro-metastatic effect of ionizing radiation (IR) has been unveiled by classical studies in animal models. Tumour progression after radiotherapy could result from positive selection of the "cancer stem cell" subpopulation, which is intrinsically radioresistant. However, striking evidence indicates that, aside from selection, IR promotes an adaptive phenotype aimed at tissue regeneration, which can turn out in metastatic behaviour. This phenotype is defined as the "stress-and-recovery" response to DNA damage, occurring both at the single cell and tissue level. In single cells, detection of DNA damage elicits specific molecular mechanisms, mostly orchestrated by the ATM-p53 axis, which block replication and activate DNA repair. If the damage is irreversible, a normal cell is programmed to execute apoptosis, or to hibernate its proliferative ability through senescence. However, after death of mutant cells, tissues must restore an adequate cell number and pattern, so as to recover the original structure and function. Thus, regeneration (or "wound healing") is initiated by surviving cells, either normal or neoplastic. As observed *in vitro,* this process includes steps such as detachment from the wound border, acquisition of a fibroblast phenotype, migration into the scratched area, and, possibly, proliferation. The entire program has been referred to as "epithelial-mesenchymal transition" (EMT), a terminology underscoring morphological features. More recently, this program has also been defined as "invasive growth" (IG), a wording that emphasizes functional aspects relevant for cancer. It is now widely accepted that EMT/IG is a physiological program for tissue development and regeneration, which is usurped by cancer cells to perform invasion and metastasis. EMT/IG is activated in cancer cells (a) sometimes, as result of genetic lesions supporting clonal selection; (b) more often, as result of an adaptive response to adverse environmental conditions.

Thus, EMT/IG is a genetic program ultimately controlled by a few specific transcription factors, and orchestrated by a handful of extracellular signals. The latter include scatter factors, such as Hepatocyte Growth Factor (HGF) and Macrophage Stimulating Protein (MSP), which bind tyrosine kinase receptors belonging to the Met family.

### OBJECT AND SUMMARY OF THE INVENTION

The need is therefore felt for improved solutions for enhancing efficacy of radiotherapy in patients suffering from tumors.

The object of this disclosure is providing such improved solutions.

According to the invention, the above object is achieved thanks to the subject matter recalled specifically in the ensuing claims, which are understood as forming an integral part of this disclosure.

An embodiment of the invention provides the use of a Met inhibitor in enhancing efficacy of radiotherapy, reducing and/or abrogating patient's resistance to radiotherapy, in the treatment of a patient suffering from a tumor, preferably a tumor presenting a deregulated Met pathway, wherein the Met inhibitor is selected from:
i) DN30 anti-Met monoclonal antibody,
ii) a genetically engineered antibody containing the six complementarity determining regions (CDRs) of the DN30 anti-Met monoclonal antibody, said CDRs having the amino acid sequences forth in SEQ ID No.: 12 to 14 and 20 to 22, and
iii) a fragment of (i) or (ii) containing the six complementarity determining regions (CDRs) of DN30 anti-Met monoclonal antibody, said CDRs having the amino acid sequences forth in SEQ ID No.: 12 to 14 and 20 to 22, wherein DN30 anti-Met monoclonal antibody is produced by the hybridoma cell line ICLC PD 05006, and wherein the Met inhibitor is able to induce down-regulation of the receptor encoded by the MET gene.

Another embodiment of the present disclosure concerns a nucleotide sequence encoding a Met inhibitor for use in enhancing efficacy of radiotherapy, reducing and/or abrogating patient's resistance to radiotherapy, in the treatment (e.g. by gene-therapy) of a patient suffering from a tumor, preferably a tumor presenting a deregulated Met pathway, said Met inhibitor being selected from:
i) DN30 anti-Met monoclonal antibody,
ii) a genetically engineered antibody containing the six complementarity determining regions (CDRs) of DN30 anti-Met monoclonal antibody, said CDRs having the amino acid sequences forth in SEQ ID No.: 12 to 14 and 20 to 22, and
iii) a fragment of (i) or (ii) containing the six complementarity determining regions (CDRs) of DN30 anti-Met monoclonal antibody, said CDRs having the amino acid sequences forth in SEQ ID No.: 12 to 14 and 20 to 22, ,
wherein DN30 anti-Met monoclonal antibody is produced by the hybridoma cell line ICLC PD 05006, and wherein said Met inhibitor is able to induce down-regulation of the receptor encoded by the *MET* gene

According to a preferred embodiment, the Met inhibitor is for administration i) in the form of soluble protein by injection or infusion or ii) by means of a vector for systemic or intra-tumor administration.

According to a further preferred embodiment, the Met inhibitor is in form of a Fab fragment optionally conjugated with at least one stabilizing molecule, wherein the stabilizing molecule is selected from polyethylenglycol, albumin binding domain, albumin.

The present disclosure discloses that irradiation upregulates *MET* expression (oncogene known to drive "invasive growth" of cancer), which in turn promotes cell invasion and protects cells from radiation-induced apoptosis. Thus, abrogation of *MET* expression or inhibition of its kinase activity by specific compounds, i.e. specific Met inhibitors, promote apoptosis and counteract radiation-induced invasiveness, thus enhancing efficacy of radiotherapy.

### BRIEF DESCRIPTION OF THE DRAWINGS

The invention will now be described, by way of example only, with reference to the enclosed figures, wherein:

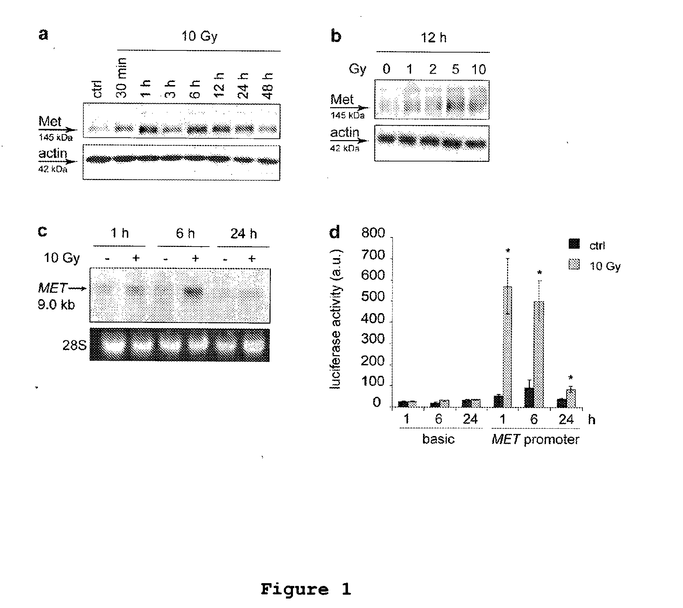

- **Figure 1****. IR induces *MET* transcription.**
   **a**, Met protein in MDA-MB-435S at the indicated time-points after irradiation (10 Gy). ctrl, Met at time zero. **b,** Met protein in MDA-MB-435S 12 h after irradiation (1-10 Gy). **c,** *MET* transcript in MDA-MB-435S at the indicated time-points after irradiation (10 Gy). **d,** Luciferase activity driven by the *MET* promoter (basic, promoterless construct) in MDA-MB-231 at the indicated time-points after irradiation (10 Gy; ctrl, non-irradiated cells). Columns: mean of triplicate analyses of two independent experiments ± s.e.m. (* p<0.05, *n* = 6, paired *t*-test). a.u., arbitrary units.

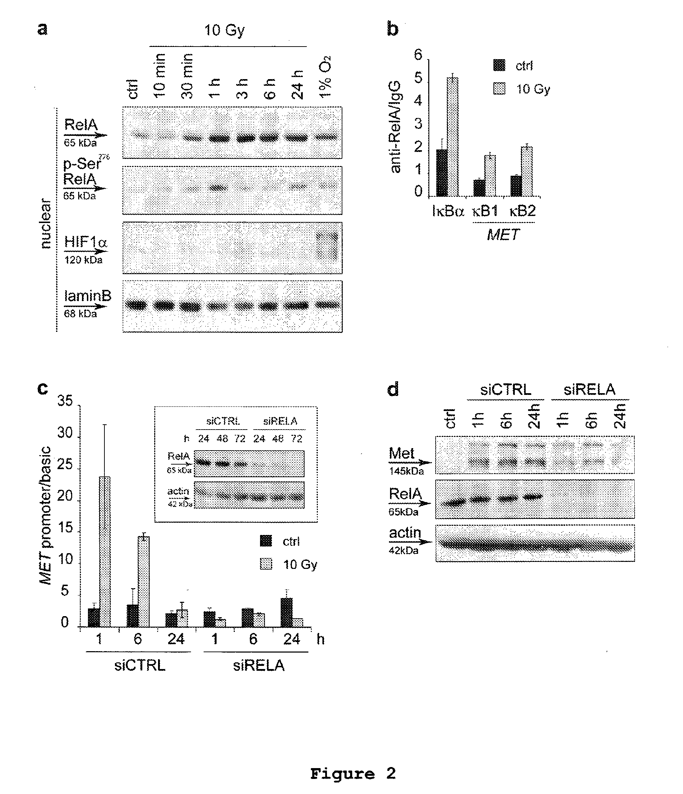

- **Figure 2****. IR-induced *MET* transcription requires NF-κB.**
   **a**, Protein nuclear accumulation in MDA-MB-435S analyzed at the indicated time-points after irradiation (10 Gy), or after 24 h culture in hypoxia (1% O₂). ctrl, non-irradiated cells at time zero. **b,** Chromatin immunoprecipitation in irradiated MDA-MB-231 (10 Gy; ctrl, non irradiated cells). Columns represent the ratio between anti-p65/RelA and nonspecific IgG immunoprecipitation of each NF-κB binding sequence (κB1 or κB2) in the *MET* promoter (mean ± s.e.m. of triplicate analyses). The *.NFKBIA* (IκBα) promoter was used as positive control. **c,** *MET* promoter activity in MDA-MB-231, silenced for p65/RelA expression (siRELA; siCTRL, control), and irradiated (10 Gy; ctrl, non-irradiated cells). Columns represent the ratio between MET promoter-driven and promoterless (basic) luciferase expression (mean of triplicate analyses in three independent experiments ± s.e.m). Inset: p65/RelA protein after siRNA transfection. **d,** Met protein accumulation in MDA-MB-435S silenced for p65/RelA expression (siRELA; siCTRL, control), at the indicated time-points after irradiation (ctrl, non-irradiated cells at time zero).
- **Figure 3****. IR-induced *MET* expression requires ATM kinase activation.**
   Met protein expression, Chk2 phosphorylation (p-Chk2) and p65/RelA nuclear translocation in MDA-MB-435S treated with the ATM kinase inhibitor CGK733, and extracted at the indicated time-points after irradiation. ctrl, non-irradiated cells at time zero.
- **Figure 4****. IR-induced invasive growth requires Met. a,** Basement membrane invasion by irradiated MDA-MB-231 or U-251 (10 Gy; ctrl, control). Micrographs of transwell filters (10X). **b,** Aberrant Met-induced branching morphogenesis in irradiated MDA-MB- 435S (10 Gy; ctrl, control), cultured with or without (-) the indicated HGF concentrations. Scale bar: 100 µm.
- **Figure 5****. Met inhibition sensitizes cells to IR-induced apoptosis and proliferative arrest.**
   Viability of U-251 irradiated with 10 Gy and/or cultured in the presence of the Fab fragment of the DN30 anti-Met antibody, for 48 h (vehicle: non-irradiated cells). Columns: mean of triplicate analyses of three independent experiments ± s.e.m. (* p<0.05, viability significantly reduced with respect to either Fab-DN30 or 10 Gy alone, *n* = 9, paired *t*-test). Columns: percentage of cells generating clones (mean of triplicate analyses of two independent experiments ± s.e.m., * p< 0.05, *n* = 6, paired *t*-test).

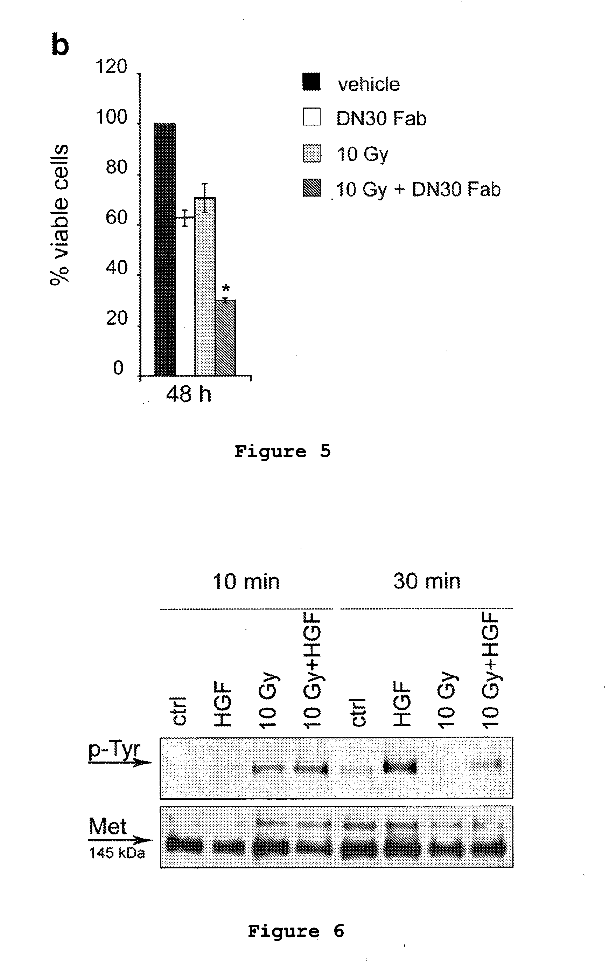

- **Figure 6****. IR induces Met phosphorylation**.
   Met phosphorylation in MDA-MB-231 treated with HGF (50 nM) and/or IR (10 Gy)Cells were immunoprecipitated with anti-Met antibodies at the indicated time-points and analyzed by western blot with anti-phospho-Tyr antibodies (p-Tyr). Met was shown as control of protein immunoprecipitation. ctrl, cells treated with HGF negative control (see Methods)

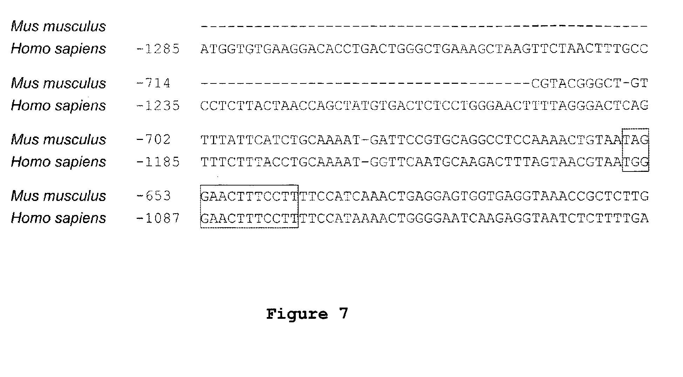

- **Figure 7** **Alignment of mouse and human *MET* promoter**.
   The human *MET* promoter (GenBank accession N°: AF046925) was analyzed with the TRANSFAC 7.0 software (Biobase Biological Database Gmbh, Wolfenbuttel, Germany) for identification of transcription factor binding sites. Two putative NF-KB binding sites (KB1 and KB2) were found. Alignment of the human and mouse (Gene ID: 17295) *MET* promoter shows that the κB2 site (-1149/- 1136 in the human sequence, rectangle) is highly conserved between the two species.

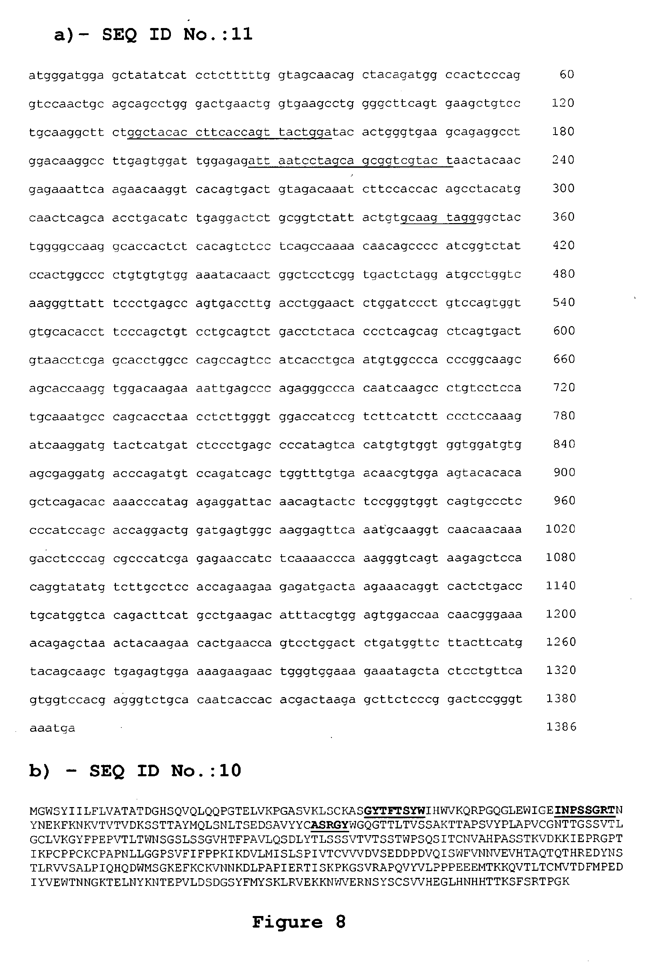

- **Figure 8****: Nucleic acid (a) and amino acid (b) sequence of DN30 monoclonal antibody heavy chain**. The CDR regions are underlined both in the nucleotide and amino acid sequences.

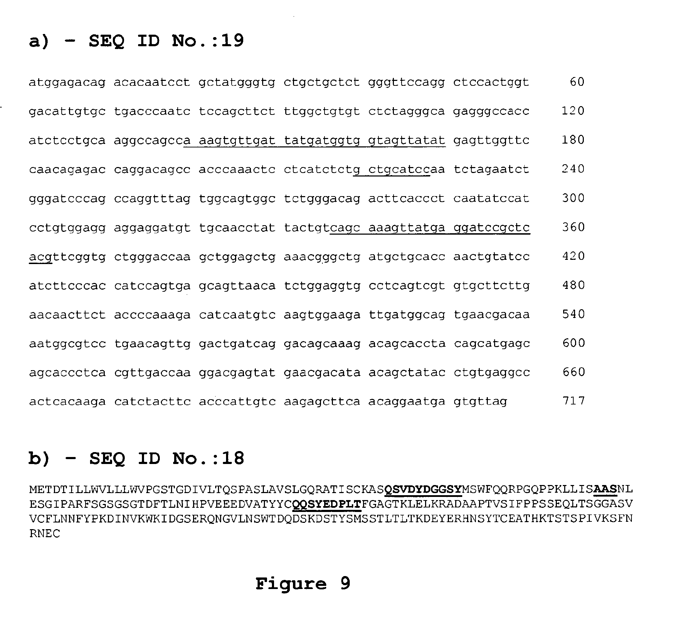

- **Figure 9****: Nucleic acid (a) and amino acid (b) sequence of DN30 monoclonal antibody light chain**. The CDR regions are underlined both in the nucleotide and amino acid sequences.

### DETAILED DESCRIPTION OF EMBODIMENTS OF THE INVENTION

The present invention will now be described in detail in relation to some preferred embodiments by way of non limiting examples.

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

The headings provided herein are for convenience only and do not interpret the scope or meaning of the embodiments.

Besides damaging intracellular targets, ionizing radiation (mostly through generation of Reactive Oxygen Species) tunes the activity of regulatory molecules, which control the stress-and-recovery biological response.

Transcriptional upregulation of the *MET* oncogene emerges as a crucial event in this response, resulting in the execution of a pro-survival and regenerative program that counteracts radiation-induced damage. This disclosure shows that IR-induced *MET* upregulation is controlled by a signal transduction pathway elicited by the protein kinase ATM following detection of DNA lesions. This pathway involves nuclear export of the ATM kinase and release of the transcription factor NF-κB from inhibition. Remarkably, it is known that activation of NF-κB by DNA damage plays a key role in the defensive response against radiation, as NF-κB is a prominent regulator of anti-apoptotic genes. It has been proposed that cell survival promoted by NF-κB is so effective as to induce "adaptive resistance" of cancer cells to radiotherapy. The present inventors now show that the adaptive response to radiation sustained by NF-KB crucially involves the *MET* proto-oncogene.

*MET* induction by IR is a biphasic transcriptional event, mediated by binding of NF-κB to the two κB specific response elements located in the *MET* promoter. The early transcriptional response occurring within 1-2 h after irradiation likely relies on activation of NF-κB by the intrinsic pathway driven by the DNA damage sensor-ATM. Conceivably, IR-induced Met overexpression is *per se* sufficient to elicit signal transduction in the presence of physiological concentrations of the ubiquitous ligand HGF, as shown in the case of hypoxia-induced Met overexpression. The late and sustained *MET* upregulation - prolonged over 24 h - is also likely to be supported by multiple extrinsic signalling pathways impinging on NF-κB. In fact, irradiation promotes expression of cytokines including TNF-α, IL-1 and IL-10 that, on one hand, are NF-κB targets, and, on the other hand, stimulate NF-κB transcriptional activity. The present inventors consider that, in living tissues, irradiation induces autocrine/paracrine loops reverberating on NF-κB that propagate waves of survival signals throughout the damaged tissue.

Remarkably, it is known that the transcriptional response to NF-κB includes, in addition to pro-survival genes, molecules responsible for EMT/IG. The combined execution of pro-survival and EMT/IG genetic programs acts as a double-edge sword: in normal tissues, these programs result in survival and regeneration after damage; in cancer cells, they foster progression towards malignancy.

The *MET* proto-oncogene meets the criteria for being a critical NF-κB target, required for orchestrating both the bright and the dark side of the stress-and-recovery responses. As it is shown in the instant disclosure, on one hand, IR-induced Met overexpression enables cells to heal wounded monolayers. On the other hand, IR stimulates cells to cross basement membranes, a typical hallmark of malignant tumours. Even more strikingly, it is reported that IR turns the physiological process of Met-induced branching morphogenesis into disorganized cell dissemination throughout a tridimensional matrix. In all cases, although several NF-κB target genes are expressed in irradiated cells, through *MET* knock-down or functional inhibition, the present inventors show that Met is required for both physiological invasive growth (wound healing) and malignant invasive growth (invasiveness). The reported aggressiveness of tumours relapsing after irradiation may, thus, involve activation of the EMT/IG program under a tight control of the *MET* oncogene.

The observation that Met is implied in the anti-apoptotic, regenerative and invasive response to IR has important therapeutic consequences: combination of radiotherapy with Met inhibition radiosensitizes cancer cells, while preventing pro-invasive collateral effects. Indeed the present disclosure shows that Met inhibition significantly impairs cell survival and clonogenic ability after exposure to therapeutic doses of IR. Most importantly, being expressed in the stem/progenitor compartment of several normal tissues, *MET* is conceivably expressed also in cancer stem cells, which often derive from direct transformation of normal stem cells or proliferating progenitors. IR-induced Met expression and activation support cancer (stem) cell radioresistance and invasive ability, thus increasing the chance of their positive selection and dissemination.

Therefore, Met inhibition (by means of administration of the Met inhibitor in form of soluble protein or by gene-therapy i.e. administration of a vector encoding the Met inhibitor as defined in the following) in combination with conventional therapies, i.e. radiotherapy, is a further strategy to eradicate cancer.

In the present disclosure with the expression "Met inhibitor" is meant an anti-Met monoclonal antibody, derivatives and/or fragments thereof able to induce down-regulation of the receptor encoded by the MET gene. In a preferred embodiment the "Met inhibitor" is DN30 anti-Met monoclonal antibody, derivatives and/or fragments thereof which are able to induce down-regulation of the receptor encoded by the MET gene

With the expression "antibody derivative" is meant a molecule containing the Complementary Determining Regions (CDRs) of the antibody, such as a genetically engineered or humanized antibody containing the CDRs of the antibody or a peptide containing the CDRs of the antibody.

With the expression "antibody fragment" is meant a fragment selected from Fv, scFv, Fab, Fab', F(ab')₂ fragments of i) the anti-Met monoclonal antibody, and ii) genetically engineered or humanized antibody containing the Complementary Determining Regions (CDRs) of the anti-Met monoclonal antibody.

From a pharmacological viewpoint, the employment of a Fab fragment has both advantages and disadvantages. Fab molecules can be easily produced using simple expression systems including lower eukaryotes and prokaryotes (Chambers RS. Curr Opin Chem Biol 2005 9:46-50). Fab molecules are also less immunogenic compared to whole antibodies and their lower molecular weight improves tissue penetration.

A problem in the use of Fab fragments in clinics relates to the short plasma half-life of Fab fragments that is due to higher kidney clearance. This can be circumvented by local administration of the Fab molecule to the tumor site. For therapeutic applications that require systemic delivery and prolonged treatment, actions aimed at incrementing Fab half-life are necessary. In order to get an incremented Fab half-life, a stabilized form of Fab obtained by conjugation with a stabilizing molecule (that does not modify the antigen binding properties of the Fab fragment) has been realized.

Although pegylation is the most consolidated technique (Chapman AP. Adv Drug Deliv Rev 2002 54:531-545.), pegylation is not the only possibility for implementing the stability of therapeutic proteins.

Alternatively to the chemical modification, the recombinant Fab molecules can be modified at the level of primary nucleotide sequence to incorporate sequences encoding peptides or domains capable to bind with high affinity the serum albumin (Dennis MS, et al., J Biol Chem 2002 277:35035-35043; Stork R, et al. Protein Eng Des Sel 2007 20:569-576) or can be generated as a chimeric molecule in which one of the chain encoding the Fab is fused in frame with a sequence encoding a protein biologically inactive (e.g. serum albumin (Subramanian GM, et al. Nat Biotechnol 2007 25:1411-1419)). Polyethylenglycol, albumin binding domain, albumin, or any other sequence that does not modify the antigen binding properties of the Fab fragment can be used as stabilizing molecules capable to increase the *in vivo* plasma half-life of the Fab fragment.

DN30 anti-cMet monoclonal antibody is produced by the hybridoma cell line deposited by Advanced Biotechnology Center (ABC), Interlab Cell Line Collection (ICLC), S.S. Banca Cellule e Colture in GMP, Largo Rosanna Benzi 10, Genova, Italy with accession number ICLC PD 05006.

Tumors suitable for administration of a Met inhibitor in order to reduce and/or abrogate radiotherapy resistance according to instant disclosure include i) carcinomas, preferably selected between bladder, breast, cholangiocarcinoma, colorectal, endometrial, esophageal, gastric, head and neck, kidney, liver, lung, nasopharyngeal, ovarian, pancreas/gall bladder, prostate, thyroid, ii) soft tissue sarcoma, preferably selected among Kaposi's Sarcoma, Leiomyosarcoma, MFH/Fibrosarcoma, iii) musculoskeletal sarcoma, preferably selected among osteosarcoma, rhabdomyosarcoma, synovial sarcoma, iv) hematopoietic malignancy, preferably selected among acute myelogenous leukemia, adult T cell leukemia, chronic myeloid leukemia, lymphomas, multiple myeloma, v) other neoplasms preferably selected among brain tumors, melanoma, mesothelioma, Wilms' tumor.

All these tumors present, in fact, a "deregulated Met pathway", wherein this expression means that these tumors are characterized by an aberrant Met signaling due to at least one of i) Met mutations, ii) Met protein overexpression, iii) Met gene amplification, iv) elevated levels of circulating HGF.

### Administration of Met inhibitors to human patients

Anti-Met antibodies will be administered through regimens similar to those adopted for antibodies targeting other receptor tyrosine kinases involved in human malignancies (e.g. Trastuzumab, an antibody against HER-2). Typically, the antibody or a derivative or fragment thereof is administered by intravenous infusion with weekly doses ranging between 5-10 mg/kg, preferably 4-8 mg/kg. For combination with radiotherapy, administration of the anti-Met antibodies will start one week, more preferably one day, before irradiation and continue until one week, preferably until 6 to 48 hours, after the end of radiotherapy.

The cDNA sequences encoding the anti-Met antibody, or derivatives or fragments thereof can be also administered to human patients through "gene therapy" procedures. The cDNA sequence is cloned in a transduction vector of viral origin (lentiviral, retroviral, adenoviral, etc.) and assembled into a viral particle, capable of specifically targeting tumor or tumor-associated cells, by means of specific surface binding proteins. The viral particle preparation is then produced in a GMP grade facility. This preparation can be either systemically or intratumorally delivered through one single or multiple injections. Tumor tissues transduced by the viral vector will express the proteins encoded by the sequences of the anti-Met antibody, or derivatives or fragments thereof thus providing an auto-inhibitory circuit.

In the following experimental data are provided; the experiments have been conducted using DN30 monoclonal antibody and/or derivatives and/or fragments thereof in order to provide a detailed description of some preferred embodiments without any limiting purpose of the instant invention.

### Materials and Methods

**Cell lines and siRNA.** Cell lines (A549, MDA-MB-231, LoVo, MDAMB- 435S, U-87MG, U-251, PC3, SF295, DLD1, SK-N-SH) were from ATCC. For ATM kinase inhibition, cells were pre-treated for 4 h before irradiation and then kept in the presence of CGK733 (10 µM in DMSO). siRNAs targeting *RELA* (ON-TARGET plus SMART pool L-003533-00 Human RELA, NM_021975, Dharmacon, 100 nM), or control siRNAs (ON-TARGET plus SMART pool, siCONTROL Non Targeting siRNA, Dharmacon) were transiently transfected.

The siRNA sequences were as follows.

"SMART pool L-003533-00 Human RELA NM_021975" was a 1:1:1:1: mixture of the following duplex sequences:
(1) sense: GGAUUGAGGAGAAACGUAAUU (SEQ ID No.:1), antisense: 5'-NUUUCCUACAAGCUCGUGGGUU (SEQ ID No.:2),
(2) sense: CCCACGAGCUUGUAGGAAAUU (SEQ ID No.:3), antisense: 5'-NUUUCCUACAAGCUCGUGGGUU (SEQ ID No.:4),
(3) sense: GGCUAUAACUCGCCUAGUGUU (SEQ ID No.:5), antisense: 5'-NCACUAGGCGAGUUAUAGCCUU (SEQ ID No.:6),
(4) sense: CCACACAACUGAGCCCAUGUU (SEQ ID No.:7), antisense: 5'-NCAUGGGCUCAGUUGUGUGGUU (SEQ ID No.:8).

SMART pool, siCONTROL Non Targeting siRNA (one single duplex sequence):
sense: AUGUAUUGGCCUGUAUUAG (SEQ ID No.:9).

**DN30 antibody and DN30 Fab fragment production.** DN30 monoclonal antibody was produced as described in Prat M. et al., 1998, J. Cell Sci 111:237-247, and deposited by Advanced Biotechnology Center with accession number ICLC PD 05006. The DN30 Fab fragment was obtained through DN30 papain digestion and affinity purification (Immunopure Fab Preparation Kit, Pierce).

The aminoacid sequence of DN30 heavy chain is illustrated in Figure 8b and set forth in SEQ ID No:10, DN30 heavy chain nucleotide sequence is illustrated in Figure 8a and set forth in SEQ ID No.:11.

The aminoacid sequences corresponding to DN30 heavy chain CDR regions are the following: CDR-H1: GYTFTSYW (SEQ ID NO.:12); CDR-H2: INPSSGRT (SEQ ID NO.:13); CDR-H3: ASRGY (SEQ ID N0.:14). The nucleotide sequences corresponding to DN30 heavy chain CDR regions are the following: CDR-H1: GGCTACACCTTCACCAGTTACTGGA (SEQ ID NO.:15); CDR-H2: ATTAATCCTAGCAGCGGTCGTACT (SEQ ID NO.:16); CDR-H3: GCAAGTAGG (SEQ ID NO.:17).

The aminoacid sequence of DN30 light chain is illustrated in Figure 9b and set forth in SEQ ID No:18, DN30 light chain nucleotide sequence is illustrated in Figure 9a and set forth in SEQ ID No.:19.

The aminoacid sequences corresponding to DN30 light chain CDR regions are the following: CDR-L1: QSVDYDGGSY (SEQ ID NO.:20); CDR-L2: AAS (SEQ ID NO.:21); CDR-L3: QQSYEDPLT (SEQ ID NO.:22). The nucleotide sequences corresponding to DN30 light chain CDR regions are the following: CDR-L1: AAAGTGTTGATTATGATGGTGGTAGTTATAT (SEQ ID NO.:23); CDR-L2: GCTGCATCC (SEQ ID NO.:24); CDR-L3: CAGCAAAGTTATGAGGATCCGCTCACG (SEQ ID NO.:25).

***In vitro* irradiation.** X-rays were emitted by a linear particle accelerator (Clinac 600C/D, Varian) operating at 6 MV.

**Western Blot.** Protein expression was investigated in irradiated confluent, serum-starved cells. For fractionation in cytoplasmic and nuclear portions, cells were washed and incubated on ice for 10 min in "buffer A" (10 mM HEPES pH 7.9, 10 mM KCl, 0.1 mM EDTA, 0.5% NP-40, 1 mM dithiothreitol, 1 mM phenylmethylsulfonyl fluoride and a cocktail of protease inhibitors). Supernatants, containing the cytoplasmic extracts, were separated by centrifugation. Pellets were resuspended in "buffer B" (20 mM HEPES pH 7.9, 400 mM KCl, 1 mM EDTA, 1 mM dithiothreitol, 10% glycerol, 1 mM phenylmethylsulfonyl fluoride and a cocktail of protease inhibitors) and incubated at 4°C for 1 h with vigorous mixing. The resulting nuclear lysates were clarified by high-speed centrifugation. Equal amount of proteins were resolved by SDS-PAGE and analysed by western blot with the following primary antibodies: mouse anti-human Met (DL21 disclosed in Prat et al., Int. J. Cancer 49, 323-328 (1991)), mouse anti-p65/RelA and mouse anti-HIF-1α (BD Biosciences), rabbit anti-phospho-Ser276 and rabbit anticaspase-3 (Cell Signaling), rabbit anti-phospho-Chk2 (T68, R&D Systems). Goat anti-actin (C-11; Santa Cruz Biotechnology) and mouse anti-lamin B (Calbiochem) were used for control of equal cytoplasmic or nuclear protein loading, respectively. Blot images were captured using a molecular imager (GelDoc XR; Bio-Rad Laboratories). Densitometric analysis was performed with Quantity One 1-D (Bio-Rad Laboratories). Western blots shown are representative of results obtained in at least three independent experiments.

**Northern Blot.** Confluent cells were serum-starved for 48 h and irradiated. Total RNA was resolved in 0.8% agarose-formaldehyde gel, and transferred to nylon membranes (Amersham) according to standard procedures. The *MET* probe containing the whole coding sequence (GenBank Accession N. J02958) was obtained from the pCEV-MET plasmid (see Michieli et al., Oncogene 18, 5221-5231 (1999)), and labelled with [α-³²P]dCTP (Megaprime, Amersham). Hybridization was carried out at 42°C for 16 h in the presence of 50% formamide. Nylon membranes were washed twice with 2X SSC-0.1% SDS, and twice with 0.1X SSC-0.1% SDS at 42°C, and autoradiographed.

**ROS detection.** Intracellular ROS generation was assessed using 5-(and 6)-carboxy-2'-7'-dichlorofluorescein diacetate (carboxy-H₂DCFDA, Molecular Probes) according to manufacturer's instructions. Briefly, cells were seeded in black 96-well plates (3 x 10⁴ cells/well) 24 h before treatment (IR: 10 Gy; H₂O₂: 100 µM as control). Cells were incubated in the presence of carboxy-H₂DCFDA (10 µM) in phenol red-free DMEM for 1 h at 37°C. Cells were washed, incubated for additional 30 min in phenol red-free DMEM without carboxy-H₂DCFDA, and then irradiated. ROS generation was measured 15 min after irradiation using a fluorescence plate reader (λₑₓ = 485 nm, λₑₘ = 535 nm) (DTX 880 Multimode Detector).

**Chromatin immunoprecipitation (ChIP).** 4 x 10⁷ cells were used for 10 ChIPs either for irradiated or control cells. After irradiation (10 Gy), cells were fixed in 1% formaldehyde for 15 min at room temperature, and reaction was stopped with glycine (0.125 M). Fixed cells were washed, collected in ice cold PBS supplemented with a cocktail of protease inhibitors and centrifuged. The cytoplasmic fraction was extracted as above and discarded, and nuclei were pelletted and resuspended in 1 ml of SDS-Lysis Buffer (1% SDS, 1 mM EDTA, 50 mM Tris-HCl pH 8, and a cocktail of protease inhibitors). Nuclei were then disrupted by sonication, yielding DNA fragments with a bulk size of 400-1000 bp. Cell debris were cleared by centrifugation at 14.000 rpm for 10 min at 4°C. The supernatants containing the chromatin preparation were diluted with Dilution Buffer 10X (0.01% SDS, 1.1% Triton X-100, 1.2 mM EDTA, 16.7 mM Tris-HCl pH 8, 167 mM NaCl). Chromatin was then pre-cleared for 1 h at 4°C by adding protein G-sepharose (Amersham, 50% gel slurry supplemented with 0.2 mg/ml of salmon sperm DNA, 0.1% BSA and 0.05% NaN₃). Beads were pelleted by a brief centrifugation at 4000 rpm at 4°C and supernatants were collected. 3% of chromatin preparation was used as Input for ChIP normalisation. ChIP was performed overnight at 4°C with 4 µg of antibodies (anti-p65/RelA, Santa Cruz; total mouse IgG, Chemicon), followed by incubation with 50 µl of protein G-sepharose beads for 3 h. Beads were washed sequentially on a rotating platform at 4°C with the following solutions (10 min/each) : twice with Low-Salt Buffer (0.1% SDS, 1% Triton X-100, 2 mM EDTA, 20 mM Tris-HCl pH 8, 150 mM NaCl), twice with High-Salt Buffer (0.1% SDS, 1% Triton X-100, 2 mM EDTA, 20 mM Tris-HCl pH 8, 500 mM NaCl), once with LiCl Buffer (0.25 M LiCl, 1% Deoxycholic Acid, 0.5% NP-40, 1 mM EDTA, 10 mM Tris-HCl pH 8), and twice with TE (10 mM Tris-HCl pH 8, 1 mM EDTA). ChIPs were eluted twice in EB (1% SDS, 0.1 M NaHCO₃) and kept overnight at 65°C to reverse formaldehyde cross-linking. Treatment with RNase (50 µg/ml, 37°C for 30 min) and Proteinase-K (500 µg/ml, 45°C for 2 h) were performed. Each sample was purified by phenol/chloroform extraction and finally resuspended in 40 µl of sterile water. 2 µl of each sample were used as template for Real-Time PCR with SYBR GREEN PCR Master Mix (Applied Biosystems) on ABI PRISM 7900HT sequence detection system (Applied Biosystems) .

Primers used were as follows:
- NFKBIA (fw: GAACCCCAGCTCAGGGTTTAG - SEQ ID No.:26; rev: GGGAATTTCCAAGCCAGTCA - SEQ ID No.:27);
- KB1 (fw: AGGCCCAGTGCCTTATTACCA - SEQ ID No.:28; rev: GCGGCCTGACTGGAGATTT - SEQ ID No.:29);
- KB2 (fw: GGGACTCAGTTTCTTTACCTGCAA - SEQ ID No.:30; rev: GGGACTCAGTTTCTTTACCTGCAA - SEQ ID No.:31).

**Wound healing assay**. Cells were grown to confluence in 24-well plates, starved for 24 hr, and scratched with a plastic tip. Culture medium was replaced with fresh medium containing 1% FBS and the vehicle alone (DMSO), and immediately irradiated. After 24 h, cells were fixed with 11% glutaraldehyde (Sigma), and stained with crystal violet. Images were acquired with a Leica photocamera (Leica DFC320, Leica) connected with an inverted light microscope (DM IRB, Leica). Images are representative of results obtained in at least three independent experiments.

**Transwell** assay. Cell invasion was measured in Transwell™ chambers (BD Falcon). MDA-MB-231 and MDA-MB-435S cells (5 x 10⁵/transwell) were seeded on the filter upper sides coated with 20 µg/cm² of reconstituted Matrigel basement membrane (Collaborative Research). U-251 (10⁴/transwell) were seeded on filters coated with 50 µg/cm². Culture medium supplemented with 1% FBS was added to both chambers. 1 h after seeding, cells were irradiated (10 Gy) and incubated at 37°C for 24 h. Cells on the filter upper side were mechanically removed, and those migrated onto the lower side were fixed, stained, and photographed as above. For quantification of cell invasion, ten fields per experimental condition were randomly selected and micrographed as above with a 10X objective. Morphometric analysis was performed using MetaMorph 7.1 software. Images are representative of at least three independent experiments.

**Branching morphogenesis assay.** MDA-MB-435S spheroids were preformed by single-cell resuspension in 240 mg/ml methylcellulose (Sigma) and culture in nonadherent 96-well plates (Greiner) for 24 h. Spheroids were transferred into a matrix containing 1.3 mg/ml type I collagen from rat tail (BD Biosciences), 10% FBS, and 240 mg/ml methylcellulose. After 24 h, cells were irradiated and/or cultured in the presence of HGF for 7 days. HGF was obtained as a baculovirus recombinant protein in SF9 cells. The conditioned medium from uninfected cells was used as negative control. Images are representative of results obtained in three independent experiments.

**Cell viability assay with DN30 Fab.** 10³ cells were seeded in 96-well plates and grown for 24 h. Culture medium was replaced with medium containing 1% FBS and DN30 Fab (28 µg/ml) or the vehicle alone (PBS). After 24 h, cells were irradiated (10 Gy). Cell viability was assessed as above.

### Results

### IR induces MET transcription

The present inventors have previously shown that the *MET* proto-oncogene is transcriptionally regulated by extra- and intracellular specific signals, including growth factors and the oxygen sensor. Here it is investigated modulation of Met expression by exposure to therapeutic doses of IR (up to 10 Gy).

In ten cell lines derived from neoplastic tissues of different histological types (carcinomas of breast, lung, prostate and colon; melanoma; glioblastoma; neuroblastoma), it has been found that the Met protein level was significantly increased 24 h after irradiation. In representative cell lines (such as MDA-MB-435S and MDA-MB-231), detailed time course experiments revealed a biphasic profile of Met protein accumulation. This is characterized by an early peak of Met induction (∼five fold) around 1-2 h, followed by a similar late peak or a plateau, appearing at 6 h, and decreasing 24 h after irradiation (Fig. 1a). Dose-response experiments showed that Met induction starts after 1 Gy, and reaches a plateau at 5 Gy (Fig. 1b). In irradiated cells, *MET* mRNA accumulation, and activation of the full-length *MET* promoter were also observed (Fig. 1c-d), indicating that IR-induced *MET* overexpression involves a transcriptional mechanism. Interestingly, in MDA-MB-231, a transient and ligand-independent Met autophosphorylation was also detected, occurring within 10 min after exposure to IR, (Fig. 6). The intensity of IR-induced Met phosphorylation was comparable to that elicited by a non-saturating concentration of HGF (50 ng/ml). However, the kinetics of phosphorylation were different, as the peak induced by IR was reached after 10 min, while the peak induced by HGF was reached after 30 min. Concomitant stimulation by IR and HGF was not synergistic (Fig. 6).

### IR-induced MET transcription requires NF-κB

IR is known to modulate a few transcription factors including NF-κB. Accordingly, genome-wide expression profiling showed that, in the cell lines examined, IR induces a prominent early NF-κB response. For instance, in MDA-MB-231, 9 out of the 33 genes modulated 1 h after irradiation are NF-κB targets, displaying a frequency -20 fold higher than expected. Moreover, in time-course experiments with MDA-MB-231, MDA-MB-435S or U-251 cells, IR (10 Gy) induced rapid (within 30 min) and persistent (until 24 h) nuclear accumulation of the NF-κB subunit p65/RelA, a hallmark of NF-κB activation (Fig. 2a). Moreover, at early time-points after irradiation, nuclear p65/RelA was transiently phosphorylated at Ser²⁷⁶ (Fig. 2a). This phosphorylation is known to be induced by Reactive Oxygen Species (ROS) via protein kinase A, and to promote p65/RelA interaction with the transcriptional coactivator CBP/p300, which is required for upregulation of a subset of early target genes. These data indicate that IR promotes functional activation of NF-κB, through nuclear accumulation and early transient phosphorylation of the p65/RelA subunit.

In the *MET* human promoter, two putative NF-κB binding sites, κB1, located at -1349/-1340 bp, and κB2, located at -1149/-1136 bp, with respect to the transcription start site of the sequence (GenBank accession No. AF046925) were identified through *in silico* analysis. Interestingly, the κB2 site is highly conserved in the *met* mouse promoter (Fig. 7; *met* mouse (mus musculus) promoter sequence set forth in SEQ ID No:32 and *met* human (homo sapiens) promoter sequence set forth in SEQ ID No.:33). Chromatin immunoprecipitation experiments showed that association of p65/RelA to either site was significantly increased in cells exposed to 10 Gy (Fig. 2b), indicating that *MET* is transcriptionally controlled by NF-κB in irradiated cells. These findings prompted the present inventors to investigate whether NF-κB is an absolute requirement for *MET* induction by IR. As p65/RelA is involved in the formation of each of the several NF-κB heterodimers, thus being critical for the entire NF-κB-driven transcriptional activity, p65/RelA expression was abrogated through RNA interference. In MDA-MB-231 or MDA-MB-435S treated with siRNA against p65/RelA (SMART pool L-003533-00 Human RELA, SEQ ID No.: 1 to 8), IR could no longer induce the full-length *MET* promoter activity (Fig. 2c), neither accumulation of the Met protein. Taken together, these data provide convincing evidence that IR-induced *MET* upregulation requires activation of the transcription factor NF-κB.

The involvement of Hypoxia Inducible Factor-1 (HIF-1) in IR-induced *MET* transcription was also considered, since (a) HIF-1 was shown to be activated in irradiated cells as result of ROS formation, and (b) HIF-1 is a prominent regulator of *MET* expression. However, the relevance of HIF-1 was minimal, as shown by complementary approaches. First, in MDA-MB-231 and MDA-MB-435S, IR did not induce nuclear translocation of the HIF-1α subunit, which is the hallmark of HIF-1 activation, otherwise observed when cells were cultured in low oxygen concentration (Fig. 2a). Lack of HIF-1 activation was not due to weak ROS production in irradiated cells, as ROS were increased by 25 ± 3.5% on average, 15 min after exposure to 10 Gy. This was estimated to correspond to an average 80% ROS induction 2-5 min after irradiation, accordingly to previous observations in cell lines exposed to 1-10 Gy. Moreover, it has been found that IR could not activate the so-called "minimal" *MET* promoter including the two functional Hypoxia Responsive Elements (HRE), and the Ap-1 site, which are responsible for hypoxia-induced *MET* upregulation. Taken together, these data indicate that HIF-1 is not involved in MET upregulation by IR. However, it has been observed that hypoxia induced p65/RelA nuclear translocation and serine phosphorylation (Fig. 2a). Finally, the involvement of the transcription factor p53, a prominent IR-target, was ruled out as well. In fact, MDA-MB-435S and MDA-MB-231 (two cell lines displaying the highest *MET* induction by IR) harbour p53 inactivating mutations (G266E and R280K, respectively). Moreover, unlike the mouse promoter, the human *MET* promoter is not upregulated by constitutively active forms of p53.

### IR-induced MET expression is mediated by ATM kinase activation

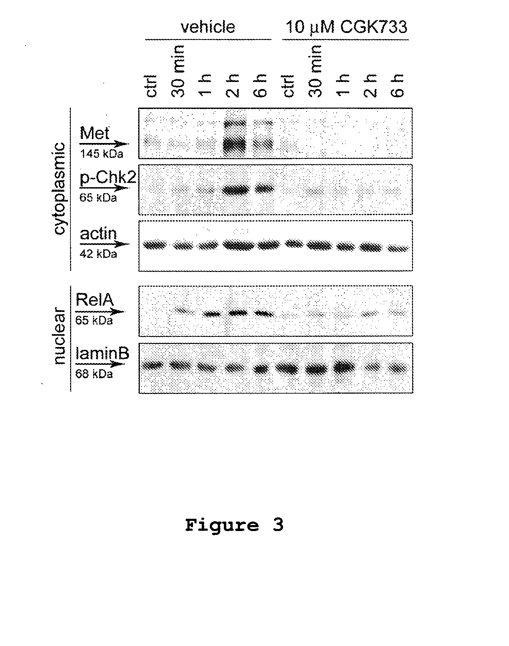

NF-κB is a crossroad of several pathways initiated both by extracellular and intracellular signals. The latter include those elicited by protein kinase ATM following detection of DNA damage. To investigate whether *MET* induction by IR relies on activation of the ATM kinase, MDA-MB-435S or MDA-MB-231 were treated with 10 µM of the specific small-molecule inhibitor CGK733. In time course experiments, CGK733 prevented IR-induced phosphorylation of the specific ATM substrate Chk2, as well as p65/RelA nuclear translocation, and Met protein overexpression. These data indicate that ATM kinase is required for IR-induced *MET* upregulation (Fig. 3).

### IR-induced invasive growth requires Met

Met overexpression does not imply kinase activation in the absence of the extracellular ligand HGF. However, it entails a significant increase in ligand-dependent signalling activity (*i.e.* sensitization). This has been observed in cells where hypoxia upregulated Met expression to a level comparable to, or lower than that induced by irradiation.

The present inventors thus investigated whether IR-induced Met overexpression could elicit or potentiate the Met-dependent biological responses. These include the physiological and pathological sides of invasive growth. In wound-healing assay, assessing the ability of the cell to regenerate injured tissues (*i.e.* physiological invasive growth), irradiated MDA-MB-231, as well as MDA-MB-435S, spontaneously performed the healing program, by detaching from the edge of the wound, and migrating throughout the scratched area. This response, monitored for 24 h, was overlapping with that stimulated by HGF, which is also known as "Scatter Factor", as it promotes cell dissociation and motility. However, the healing response elicited by IR was not due to induction of a HGF autocrine loop, as irradiated cells did not express HGF as assessed by quantitative PCR. The present inventors conclude that IR-induced Met overexpression sensitizes cells to the small amount of HGF present in the culture medium, which was supplied with 1% serum. This condition likely mimics the physiological presence of HGF *in vivo,* which is ubiquitously embedded in extracellular matrices.

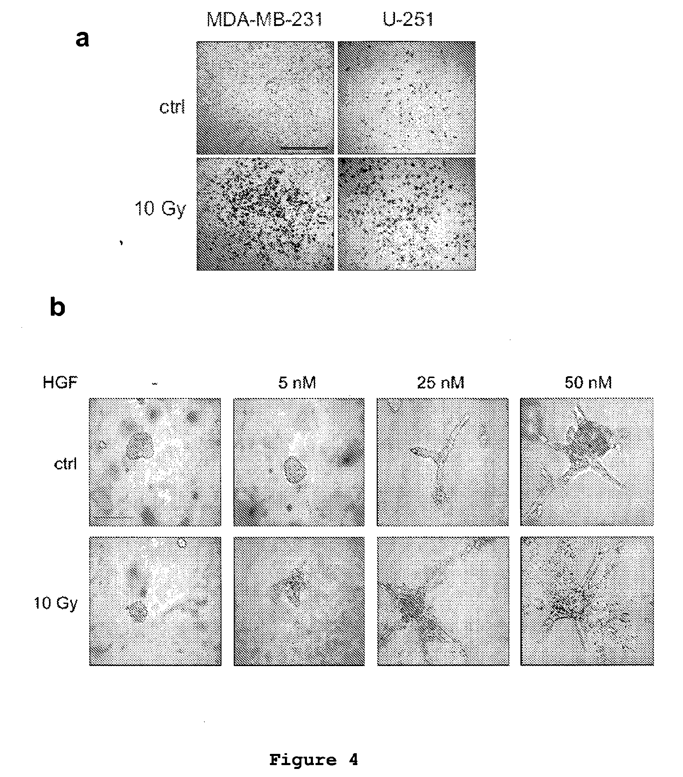

Irradiated cells were then assessed in transwell assays, measuring the ability to trespass an artificial basement membrane *in vitro,* which tightly correlates with *in vivo* invasiveness, i.e. malignant invasive growth. Indeed, irradiated cells (such as MDA-MB-231, MDA-MB-435S, or U-251) spontaneously crossed the transwell basement membrane in the presence of a low serum concentration (1%) (Fig. 4a), again mimicking the behaviour evoked by HGF.

### IR turns Met-induced morphogenesis into an invasive process

Branching morphogenesis is a complex physiological process, induced by HGF as to generate tridimensional organs during development. This multistep program entails cell migration, proliferation and spatial reorganization, ending up with generation of hollow branched tubules lined by polarized cells. Some of the cell lines studied, such as MDA-MB-435S, can fully execute the branching morphogenesis program *in vitro.*

Exposure to IR sensitized these cells to a suboptimal concentration of exogenous HGF (5 nM) that - alone - is incapable of inducing branching morphogenesis (Fig. 4b). Importantly, irradiated cells stimulated with HGF built tubules with remarkable structural alterations, as cells disengaged from the abluminal surface and spread into the surrounding matrix (Fig. 4b). This behaviour is reminiscent of the "tridimensional scatter" described as a form of aberrant morphogenesis occurring in response to TNFα. It was concluded that therapeutic doses of IR may turn physiological branching morphogenesis into an aberrant pro-invasive process.

### Met inhibition sensitizes cells to IR-induced apoptosis and proliferative arrest

As part of the EMT/IG program, Met emanates powerful anti-apoptotic signals through sustained activation of downstream pathways including PI3-kinase/AKT. The present inventors thus reasoned that *MET* upregulation could prevent cell death induced by irradiation, and that, conversely, Met inhibition could increase the efficacy of radiotherapy.

A cell viability decrease (up to 75%) was observed in irradiated cells that were kept in the presence of the Fab fragment of the DN30 anti-Met antibody, which is known to induce MET down-regulation, thus inhibiting MET signalling and biological activities (Petrelli et al., PNAS 103: 5090-5, 2006) (Fig. 5).

These results indicate that Met inhibition activity sensitizes cells to radiotherapy, by increasing cell death and reducing the ability to resume proliferation after treatment.

## Claims

1. Met inhibitor for use in enhancing efficacy of radiotherapy, reducing and/or abrogating patient's resistance to said radiotherapy, in the treatment of a patient suffering from a tumor, said Met inhibitor being selected from:
i) DN30 anti-Met monoclonal antibody,
ii) a genetically engineered antibody containing the six complementarity determining regions (CDRs) of DN30 anti-Met monoclonal antibody, said CDRs having the amino acid sequences set forth in SEQ ID NO.: 12 to 14 and 20 to 22, and
iii) a fragment of (i) or (ii) containing the six complementarity determining regions (CDRs) of DN30 anti-Met monoclonal antibody, said CDRs having the amino acid sequences set forth in SEQ ID NO.: 12 to 14 and 20 to 22,
wherein said DN30 anti-Met monoclonal antibody is produced by the hybridoma cell line ICLC PD 05006, wherein said Met inhibitor is able to induce down-regulation of the receptor encoded by the *MET* gene and to counteract radiation-induced tumor invasiveness.

2. Nucleotide sequence encoding a Met inhibitor for use in enhancing efficacy of radiotherapy, reducing and/or abrogating patient's resistance to said radiotherapy, in the treatment of a patient suffering from a tumor, said Met inhibitor being selected from:
i) DN30 anti-Met monoclonal antibody,
ii) a genetically engineered antibody containing the six complementarity determining regions (CDRs) of DN30 anti-Met monoclonal antibody, said CDRs having the amino acid sequences set forth in SEQ ID NO.: 12 to 14 and 20 to 22, and
iii) a fragment of (i) or (ii) containing the six complementarity determining regions (CDRs) of DN30 anti-Met monoclonal antibody, said CDRs having the amino acid sequences set forth in SEQ ID NO.: 12 to 14 and 20 to 22,
wherein said DN30 anti-Met monoclonal antibody is produced by the hybridoma cell line ICLC PD 05006, wherein said Met inhibitor is able to induce down-regulation of the receptor encoded by the *MET* gene and to counteract radiation-induced tumor invasiveness.

3. Met inhibitor according to claim 1, wherein said Met inhibitor is for administration in the form of soluble protein by injection or infusion.

4. Nucleotide sequence encoding said Met inhibitor according to claim 2, wherein said nucleotide sequence encoding said Met inhibitor is for administration by means of a vector, wherein said vector is in form of a particle.

5. Nucleotide sequence encoding said Met inhibitor according to claim 4, wherein said vector is suitable for targeting tumor or tumor-associated cells.

6. Nucleotide sequence encoding said Met inhibitor according to claim 4 or claim 5, wherein said vector is for systemic or intra-tumor administration, preferably by injection.

7. Met inhibitor or nucleotide sequence encoding a Met inhibitor according to any one of the preceding claims, wherein said fragment is a Fab fragment, preferably a Fab fragment comprising at least one stabilizing molecule.

8. Met inhibitor or nucleotide sequence encoding a Met inhibitor according to claim 7, wherein said at least one stabilizing molecule is selected from polyethylenglycol, albumin binding domain, albumin.

9. Met inhibitor or nucleotide sequence encoding a Met inhibitor according to any one of the preceding claims, wherein said Met inhibitor and/or said nucleotide sequence encoding said Met inhibitor is for administration at least one week before subjecting said patient to radiotherapy.

10. Met inhibitor or nucleotide sequence encoding a Met inhibitor according to any one of the preceding claims, wherein said Met inhibitor and/or said nucleotide sequence encoding said Met inhibitor is for administration one day before subjecting said patient to radiotherapy.

11. Met inhibitor or nucleotide sequence encoding a Met inhibitor according to any one of the preceding claims, wherein said Met inhibitor and/or said nucleotide sequence encoding said Met inhibitor is for administration until at least one week, preferably 6 to 48 hours, after the end of radiotherapy.

12. Met inhibitor or nucleotide sequence encoding a Met inhibitor according to any one of the preceding claims, wherein said tumor is selected among a carcinoma, a musculoskeletal sarcoma, a soft tissue sarcoma, a hematopoietic malignancy, a brain tumor, melanoma, mesothelioma, Wilms' tumor.

## Patentansprüche

1. Met-Inhibitor zur Verwendung bei der Verstärkung der Wirksamkeit von Strahlentherapie, der Reduzierung und/oder der Aufhebung der Resistenz eines Patienten gegenüber der Strahlentherapie, bei der Behandlung eines Patienten, der unter einem Tumor leidet, wobei der Met-Inhibitor ausgewählt ist aus:
i) DN30-anti-Met monoclonalem Antikörper,
ii) einem gentechnisch veränderten Antikörper, der die sechs Komplementarität-bestimmenden Regionen (CDRs) des DN30-anti-Met monoclonalen Antikörpers enthält, wobei die CDRs die in SEQ ID NO:12 bis 14 und 20 bis 22 dargestellten Aminosäuresequenzen haben, und
iii) einem Fragment aus (i) oder (ii), das die sechs Komplementaritätbestimmenden Regionen (CDRs) des DN30-anti-Met monoclonalen Antikörpers enthält, wobei die CDRs die in SEQ ID NO:12 bis 14 und 20 bis 22 dargestellten Aminosäuresequenzen haben,
wobei der DN30-anti-Met monoclonale Antikörper durch die Hybridomazelllinie ICLC-PD-05006 hergestellt wird, wobei der Met-Inhibitor in der Lage ist, die Herunterregulierung des Rezeptors, der durch das *MET*-Gen codiert wird, zu induzieren, und strahlungsinduzierter Tumorinvasivität entegenzuwirken.

2. Nucleotidsequenz, die einen Met-Inhibitor codiert, zur Verwendung bei der Verstärkung der Wirksamkeit von Strahlentherapie, der Reduzierung und/oder der Aufhebung von Resistenz eines Patienten gegenüber Strahlentherapie, bei der Behandlung eines Patienten, der unter einem Tumor leidet, wobei der Met-Inhibitor ausgewählt ist aus:
i) DN30-anti-Met monoclonalem Antikörper,
ii) einem gentechnisch veränderten Antikörper, der die sechs Komplementarität-bestimmenden Regionen (CDRs) des DN30-anti-Met monoclonalen Antikörpers enthält, wobei die CDRs die in SEQ ID NO:12 bis 14 und 20 bis 22 dargestellten Aminosäuresequenzen haben, und
iii) einem Fragment aus (i) oder (ii), das die sechs Komplementaritätbestimmenden Regionen (CDRs) des DN30-anti-Met monoclonalen Antikörpers enthält, wobei die CDRs die in SEQ ID NO:12 bis 14 und 20 bis 22 dargestellten Aminosäuresequenzen haben,
wobei der DN30-anti-Met monoclonale Antikörper durch die Hybridomazelllinie ICLC-PD-05006 produziert wird, wobei der Met-Inhibitor in der Lage ist, die Herunterregulierung des Rezeptors, der durch das *MET*-Gen codiert wird, zu induzieren, und strahlungsinduzierter Tumorinvasivität entgegenzuwirken.

3. Met-Inhibitor nach Anspruch 1, wobei der Met-Inhibitor zur Verabreichung in der Form eines löslichen Proteins durch Injektion oder Infusion ist.

4. Nucleotidsequenz, die den Met-Inhibitor nach Anspruch 2 codiert, wobei die Nucleotidsequenz, die den Met-Inhibitor codiert, zur Verabreichung mittels eines Vektors ist, wobei der Vektor in der Form eines Partikels ist.

5. Nucleotidsequenz, die den Met-Inhibitor nach Anspruch 4 codiert, wobei der Vektor geeignet ist, auf Tumorzellen oder tumorassoziierte Zellen abzuzielen.

6. Nucleotidsequenz, die den Met-Inhibitor nach Anspruch 4 oder Anspruch 5 codiert, wobei der Vektor für systemische oder intra-tumorale Verabreichung ist, bevorzugt durch Injektion.

7. Met-Inhibitor oder Nucleotidsequenz, die einen Met-Inhibitor nach einem der vorangehenden Ansprüche codiert, wobei das Fragment ein Fab-Fragment ist, bevorzugt ein Fab-Fragment, das mindestens ein stabilisierendes Molekül umfasst.

8. Met-Inhibitor oder Nucleotidsequenz, die einen Met-Inhibitor nach Anspruch 7 codiert, wobei das mindestens eine stabilisierende Molekül ausgewählt ist aus Polyethylenglykol, Albuminbindedomäne, Albumin.

9. Met-Inhibitor oder Nucleotidsequenz, die einen Met-Inhibitor nach einem der vorangehenden Ansprüche codiert, wobei der Met-Inhibitor und/oder die Nucleotidsequenz, die den Met-Inhibitor codiert, zur Verabreichung mindestens eine Woche vor dem Tag vorgesehen ist, bevor der Patient einer Strahlentherapie ausgesetzt wird.

10. Met-Inhibitor oder Nucleotidsequenz, die einen Met-Inhibitor nach einem der vorangehenden Ansprüche codiert, wobei der Met-Inhibitor und/oder die Nucleotidsequenz, die den Met-Inhibitor codiert, zur Verabreichung einen Tag vor dem Tag vorgesehen ist, an dem der Patient einer Strahlentherapie ausgesetzt wird.

11. Met-Inhibitor oder Nucleotidsequenz, die einen Met-Inhibitor nach einem der vorangehenden Ansprüche codiert, wobei der Met-Inhibitor und/oder die Nucleotidsequenz, die den Met-Inhibitor codiert, zur Verabreichung bis mindestens eine Woche, bevorzugt 6 bis 48 Stunden, nach dem Ende der Strahlentherapie ist.

12. Met-Inhibitor oder Nucleotidsequenz, die einen Met-Inhibitor nach einem der vorangehenden Ansprüche codiert, wobei der Tumor ausgewählt ist aus einem Karzinom, einem Muskel-Skelett-Sarkom, einem Weichgewebesarkom, einem hematopoetischen Malignom, einem Hirntumor, Melanom, Mesotheliom, Wilms-Tumor.

## Revendications

1. Inhibiteur de Met destiné à être utilisé pour augmenter l'efficacité d'une radiothérapie, réduire et/ou supprimer la résistance du patient à ladite radiothérapie, dans le traitement d'un patient souffrant d'une tumeur, ledit inhibiteur de Met étant choisi parmi :
i) un anticorps monoclonal anti-Met DN30,
ii) un anticorps génétiquement modifié contenant les six régions de détermination de complémentarité (CDRs) d'un anticorps monoclonal anti-Met DN30, lesdites CDRs ayant les séquences d'acides aminés présentées dans les séquences SEQ ID NO. : 12 à 14 et 20 à 22, et
iii) un fragment de (i) ou (ii) contenant les six régions de détermination de complémentarité (CDRs) d'un anticorps monoclonal anti-Met DN30, lesdites CDRs ayant les séquences d'acides aminés présentées dans les séquences SEQ ID NO.: 12 à 14 et 20 à 22,
où ledit anticorps monoclonal anti-Met DN30 est produit par la lignée cellulaire d'hybridome ICLC PD 05006, où ledit inhibiteur de Met est capable d'induire une régulation négative du récepteur codé par le gène MET et de neutraliser le pouvoir invasif d'une tumeur induite par rayonnement.

2. Séquence de nucléotides codant pour un inhibiteur de Met destinée à être utilisée pour augmenter l'efficacité d'une radiothérapie, réduire et/ou supprimer la résistance du patient à ladite radiothérapie, dans le traitement d'un patient souffrant d'une tumeur, ledit inhibiteur de Met étant choisi parmi :
i) un anticorps monoclonal anti-Met DN30,
ii) un anticorps génétiquement modifié contenant les six régions de détermination de complémentarité (CDRs) d'un anticorps monoclonal anti-Met DN30, lesdites CDRs ayant les séquences d'acides aminés présentées dans les séquences SEQ ID NO. : 12 à 14 et 20 à 22, et
iii) un fragment de (i) ou (ii) contenant les six régions de détermination de complémentarité (CDRs) d'un anticorps monoclonal anti-Met DN30, lesdites CDRs ayant les séquences d'acides aminés présentées dans les séquences SEQ ID NO.: 12 à 14 et 20 à 22,
où ledit anticorps monoclonal anti-Met DN30 est produit par la lignée cellulaire d'hybridome ICLC PD 05006, où ledit inhibiteur de Met est capable d'induire une régulation négative du récepteur codé par le gène MET et de neutraliser le pouvoir invasif d'une tumeur induite par rayonnement.

3. Inhibiteur de Met selon la revendication 1, dans lequel ledit inhibiteur de Met est destiné à une administration sous la forme d'une protéine soluble par injection ou par perfusion.

4. Séquence de nucléotides codant pour ledit inhibiteur de Met selon la revendication 2, dans laquelle ladite séquence de nucléotides codant pour ledit inhibiteur de Met est destinée à une administration au moyen d'un vecteur, où ledit vecteur est sous la forme d'une particule.

5. Séquence de nucléotides codant pour ledit inhibiteur de Met selon la revendication 4, dans laquelle ledit vecteur est approprié pour un ciblage d'une tumeur ou de cellules associées à une tumeur.

6. Séquence de nucléotides codant pour ledit inhibiteur de Met selon la revendication 4 ou la revendication 5, dans laquelle ledit vecteur est destiné à une administration par voie systémique ou intra-tumorale, de préférence par injection.

7. Inhibiteur de Met ou séquence de nucléotides codant pour un inhibiteur de Met selon l'une quelconque des revendications précédentes, où ledit fragment est un fragment Fab, de préférence un fragment Fab comprenant au moins une molécule de stabilisation.

8. Inhibiteur de Met ou séquence de nucléotides codant pour un inhibiteur de Met selon la revendication 7, où ladite au moins une molécule de stabilisation est choisie parmi du polyéthylène glycol, un domaine de liaison à l'albumine, une albumine.

9. Inhibiteur de Met ou séquence de nucléotides codant pour un inhibiteur de Met selon l'une quelconque des revendications précédentes, où ledit inhibiteur de Met et/ou ladite séquence de nucléotides codant pour ledit inhibiteur de Met est/sont destiné(e)(s) à une administration effectuée au moins une semaine avant de soumettre ledit patient à une radiothérapie.

10. Inhibiteur de Met ou séquence de nucléotides codant pour un inhibiteur de Met selon l'une quelconque des revendications précédentes, où ledit inhibiteur de Met et/ou ladite séquence de nucléotides codant pour ledit inhibiteur de Met est/sont destiné(e)(s) à une administration effectuée un jour avant de soumettre ledit patient à une radiothérapie.

11. Inhibiteur de Met ou séquence de nucléotides codant pour un inhibiteur de Met selon l'une quelconque des revendications précédentes, où ledit inhibiteur de Met et/ou ladite séquence de nucléotides codant pour ledit inhibiteur de Met est/sont destiné(e)(s) à une administration effectuée jusqu'à une semaine au moins, de préférence 6 à 48 heures, après la fin d'une radiothérapie.

12. Inhibiteur de Met ou séquence de nucléotides codant pour un inhibiteur de Met selon l'une quelconque des revendications précédentes, où ladite tumeur est choisie parmi un carcinome, un sarcome musculosquelettique, un sarcome des tissus mous, une tumeur maligne hématopoïétique, une tumeur du cerveau, un mélanome, un mésothéliome, une tumeur de Wilms.


### SEQUENCE LISTING

<110> Metheresis Translational Research SA
<120> MET inhibitors for enhancing radiotherapy efficacy
<130> BEP14021-CF
<160> 33
<170> PatentIn version 3.5
<210> 1
   <211> 21
   <212> RNA
   <213> artificial
<220>
   <223> siRNA 1 against p65/ReIA
<400> 1
   ggauugagga gaaacguaau u 21
<210> 2
   <211> 22
   <212> RNA
   <213> artificial
<220>
   <223> siRNA 2 rev. against p65/ReIA
<220>
   <221> misc_feature
   <222> (1)..(1)
   <223> n is a, c, g, or u
<400> 2
   nuuuccuaca agcucguggg uu 22
<210> 3
   <211> 21
   <212> RNA
   <213> artificial
<220>
   <223> siRNA 3 - against p65/ReIA
<400> 3
   cccacgagcu uguaggaaau u 21
<210> 4
   <211> 22
   <212> RNA
   <213> artificial
<220>
   <223> siRNA 4 - rev - against p65/ReIA
<220>
   <221> misc_feature
   <222> (1)..(1)
   <223> n is a, c, g, or u
<400> 4
   nuuuccuaca agcucguggg uu 22
<210> 5
   <211> 21
   <212> RNA
   <213> artificial
<220>
   <223> siRNA 5 - against p65/ReIA
<400> 5
   ggcuauaacu cgccuagugu u 21
<210> 6
   <211> 22
   <212> RNA
   <213> artificial
<220>
   <223> siRNA 6 - rev. against p65/ReIA
<220>
   <221> misc_feature
   <222> (1)..(1)
   <223> n is a, c, g, or u
<400> 6
   ncacuaggcg aguuauagcc uu 22
<210> 7
   <211> 21
   <212> RNA
   <213> artificial
<220>
   <223> siRNA 7 - against p65/ReIA
<400> 7
   ccacacaacu gagcccaugu u 21
<210> 8
   <211> 22
   <212> RNA
   <213> artificial
<220>
   <223> siRNA 8 - rev- against p65/ReIA
<220>
   <221> misc_feature
   <222> (1)..(1)
   <223> n is a, c, g, or u
<400> 8
   ncaugggcuc aguugugugg uu 22
<210> 9
   <211> 19
   <212> RNA
   <213> artificial
<220>
   <223> siRNA - control sequence
<400> 9
   auguauuggc cuguauuag 19
<210> 10
   <211> 461
   <212> PRT
   <213> artificial
<220>
   <223> DN30 heavy chain
<400> 10
   Met Gly Trp Ser Tyr Ile Ile Leu Phe Leu Val Ala Thr Ala Thr Asp
   1               5                   10                  15
   Gly His Ser Gln Val Gln Leu Gln Gln Pro Gly Thr Glu Leu Val Lys
               20                  25                  30
   Pro Gly Ala Ser Val Lys Leu Ser Cys Lys Ala Ser Gly Tyr Thr Phe
           35                  40                  45
   Thr Ser Tyr Trp Ile His Trp Val Lys Gln Arg Pro Gly Gln Gly Leu
       50                  55                  60
   Glu Trp Ile Gly Glu Ile Asn Pro Ser Ser Gly Arg Thr Asn Tyr Asn
   65                  70                  75                  80
   Glu Lys Phe Lys Asn Lys Val Thr Val Thr Val Asp Lys Ser Ser Thr
                   85                  90                  95
   Thr Ala Tyr Met Gln Leu Ser Asn Leu Thr Ser Glu Asp Ser Ala Val
               100                 105                 110
   Tyr Tyr Cys Ala Ser Arg Gly Tyr Trp Gly Gln Gly Thr Thr Leu Thr
           115                 120                 125
   Val Ser Ser Ala Lys Thr Thr Ala Pro Ser Val Tyr Pro Leu Ala Pro
       130                 135                 140
   Val Cys Gly Asn Thr Thr Gly Ser Ser Val Thr Leu Gly Cys Leu Val
   145                 150                 155                 160
   Lys Gly Tyr Phe Pro Glu Pro Val Thr Leu Thr Trp Asn Ser Gly Ser
                   165                 170                 175
   Leu Ser Ser Gly Val His Thr Phe Pro Ala Val Leu Gln Ser Asp Leu
               180                 185                 190
   Tyr Thr Leu Ser Ser Ser Val Thr Val Thr Ser Ser Thr Trp Pro Ser
           195                 200                 205
   Gln Ser Ile Thr Cys Asn Val Ala His Pro Ala Ser Ser Thr Lys Val
       210                 215                 220
   Asp Lys Lys Ile Glu Pro Arg Gly Pro Thr Ile Lys Pro Cys Pro Pro
   225                 230                 235                 240
   Cys Lys Cys Pro Ala Pro Asn Leu Leu Gly Gly Pro Ser Val Phe Ile
                   245                 250                 255
   Phe Pro Pro Lys Ile Lys Asp Val Leu Met Ile Ser Leu Ser Pro Ile
               260                 265                 270
   Val Thr Cys Val Val Val Asp Val Ser Glu Asp Asp Pro Asp Val Gln
           275                 280                 285
   Ile Ser Trp Phe Val Asn Asn Val Glu Val His Thr Ala Gln Thr Gln
       290                 295                 300
   Thr His Arg Glu Asp Tyr Asn Ser Thr Leu Arg Val Val Ser Ala Leu
   305                 310                 315                 320
   Pro Ile Gln His Gln Asp Trp Met Ser Gly Lys Glu Phe Lys Cys Lys
                   325                 330                 335
   Val Asn Asn Lys Asp Leu Pro Ala Pro Ile Glu Arg Thr Ile Ser Lys
               340                 345                 350
   Pro Lys Gly Ser Val Arg Ala Pro Gln Val Tyr Val Leu Pro Pro Pro
           355                 360                 365
   Glu Glu Glu Met Thr Lys Lys Gln Val Thr Leu Thr Cys Met Val Thr
       370                 375                 380
   Asp Phe Met Pro Glu Asp Ile Tyr Val Glu Trp Thr Asn Asn Gly Lys
   385                 390                 395                 400
   Thr Glu Leu Asn Tyr Lys Asn Thr Glu Pro Val Leu Asp Ser Asp Gly
                   405                 410                 415
   Ser Tyr Phe Met Tyr Ser Lys Leu Arg Val Glu Lys Lys Asn Trp Val
               420                 425                 430
   Glu Arg Asn Ser Tyr Ser Cys Ser Val Val His Glu Gly Leu His Asn
           435                 440                 445
   His His Thr Thr Lys Ser Phe Ser Arg Thr Pro Gly Lys
       450                 455                 460
<210> 11
   <211> 1386
   <212> DNA
   <213> artificial
<220>
   <223> DN30 - heavy chain
<400> 11
   atgggatgga gctatatcat cctctttttg gtagcaacag ctacagatgg ccactcccag        60
   gtccaactgc agcagcctgg gactgaactg gtgaagcctg gggcttcagt gaagctgtcc       120
   tgcaaggctt ctggctacac cttcaccagt tactggatac actgggtgaa gcagaggcct       180
   ggacaaggcc ttgagtggat tggagagatt aatcctagca gcggtcgtac taactacaac       240
   gagaaattca agaacaaggt cacagtgact gtagacaaat cttccaccac agcctacatg       300
   caactcagca acctgacatc tgaggactct gcggtctatt actgtgcaag taggggctac       360
   tggggccaag gcaccactct cacagtctcc tcagccaaaa caacagcccc atcggtctat       420
   ccactggccc ctgtgtgtgg aaatacaact ggctcctcgg tgactctagg atgcctggtc       480
   aagggttatt tccctgagcc agtgaccttg acctggaact ctggatccct gtccagtggt       540
   gtgcacacct tcccagctgt cctgcagtct gacctctaca ccctcagcag ctcagtgact       600
   gtaacctcga gcacctggcc cagccagtcc atcacctgca atgtggccca cccggcaagc       660
   agcaccaagg tggacaagaa aattgagccc agagggccca caatcaagcc ctgtcctcca       720
   tgcaaatgcc cagcacctaa cctcttgggt ggaccatccg tcttcatctt ccctccaaag       780
   atcaaggatg tactcatgat ctccctgagc cccatagtca catgtgtggt ggtggatgtg       840
   agcgaggatg acccagatgt ccagatcagc tggtttgtga acaacgtgga agtacacaca       900
   gctcagacac aaacccatag agaggattac aacagtactc tccgggtggt cagtgccctc       960
   cccatccagc accaggactg gatgagtggc aaggagttca aatgcaaggt caacaacaaa      1020
   gacctcccag cgcccatcga gagaaccatc tcaaaaccca aagggtcagt aagagctcca      1080
   caggtatatg tcttgcctcc accagaagaa gagatgacta agaaacaggt cactctgacc      1140
   tgcatggtca cagacttcat gcctgaagac atttacgtgg agtggaccaa caacgggaaa      1200
   acagagctaa actacaagaa cactgaacca gtcctggact ctgatggttc ttacttcatg      1260
   tacagcaagc tgagagtgga aaagaagaac tgggtggaaa gaaatagcta ctcctgttca      1320
   gtggtccacg agggtctgca caatcaccac acgactaaga gcttctcccg gactccgggt      1380
   aaatga                                                                 1386
<210> 12
   <211> 8
   <212> PRT
   <213> artificial
<220>
   <223> DN30 heavy chain - CDR-H1
<400> 12
   Gly Tyr Thr Phe Thr Ser Tyr Trp
   1               5
<210> 13
   <211> 8
   <212> PRT
   <213> artificial
<220>
   <223> DN30 heavy chain - CDR-H2
<400> 13
   Ile Asn Pro Ser Ser Gly Arg Thr
   1 5
<210> 14
   <211> 5
   <212> PRT
   <213> artificial
<220>
   <223> DN30 heavy chain - CDR-H3
<400> 14
   Ala Ser Arg Gly Tyr
   1 5
<210> 15
   <211> 25
   <212> DNA
   <213> artificial
<220>
   <223> CDR-H1 nucleotide
<400> 15
   ggctacacct tcaccagtta ctgga 25
<210> 16
   <211> 24
   <212> DNA
   <213> artificial
<220>
   <223> CDR-H2 nucleotide
<400> 16
   attaatccta gcagcggtcg tact 24
<210> 17
   <211> 9
   <212> DNA
   <213> artificial
<220>
   <223> CDR-H3 nucleotide
<400> 17
   gcaagtagg 9
<210> 18
   <211> 238
   <212> PRT
   <213> artificial
<220>
   <223> DN30 light chain
<400> 18
   Met Glu Thr Asp Thr Ile Leu Leu Trp Val Leu Leu Leu Trp Val Pro
   1               5                   10                  15
   Gly Ser Thr Gly Asp Ile Val Leu Thr Gln Ser Pro Ala Ser Leu Ala
   20 25 30
   Val Ser Leu Gly Gln Arg Ala Thr Ile Ser Cys Lys Ala Ser Gln Ser
   35 40 45
   Val Asp Tyr Asp Gly Gly Ser Tyr Met Ser Trp Phe Gln Gln Arg Pro
   50 55 60
   Gly Gln Pro Pro Lys Leu Leu Ile Ser Ala Ala Ser Asn Leu Glu Ser
   65 70 75 80
   Gly Ile Pro Ala Arg Phe Ser Gly Ser Gly Ser Gly Thr Asp Phe Thr
   85 90 95
   Leu Asn Ile His Pro Val Glu Glu Glu Asp Val Ala Thr Tyr Tyr Cys
   100 105 110
   Gln Gln Ser Tyr Glu Asp Pro Leu Thr Phe Gly Ala Gly Thr Lys Leu
   115 120 125
   Glu Leu Lys Arg Ala Asp Ala Ala Pro Thr Val Ser Ile Phe Pro Pro
   130 135 140
   Ser Ser Glu Gln Leu Thr Ser Gly Gly Ala Ser Val Val Cys Phe Leu
   145 150 155 160
   Asn Asn Phe Tyr Pro Lys Asp Ile Asn Val Lys Trp Lys Ile Asp Gly
   165 170 175
   Ser Glu Arg Gln Asn Gly Val Leu Asn Ser Trp Thr Asp Gln Asp Ser
   180 185 190
   Lys Asp Ser Thr Tyr Ser Met Ser Ser Thr Leu Thr Leu Thr Lys Asp
   195 200 205
   Glu Tyr Glu Arg His Asn Ser Tyr Thr Cys Glu Ala Thr His Lys Thr
   210 215 220
   Ser Thr Ser Pro Ile Val Lys Ser Phe Asn Arg Asn Glu Cys
   225 230 235
<210> 19
   <211> 717
   <212> DNA
   <213> artificial
<220>
   <223> DN30 light chain
<400> 19
   atggagacag acacaatcct gctatgggtg ctgctgctct gggttccagg ctccactggt       60
   gacattgtgc tgacccaatc tccagcttct ttggctgtgt ctctagggca gagggccacc      120
   atctcctgca aggccagcca aagtgttgat tatgatggtg gtagttatat gagttggttc      180
   caacagagac caggacagcc acccaaactc ctcatctctg ctgcatccaa tctagaatct      240
   gggatcccag ccaggtttag tggcagtggc tctgggacag acttcaccct caatatccat      300
   cctgtggagg aggaggatgt tgcaacctat tactgtcagc aaagttatga ggatccgctc      360
   acgttcggtg ctgggaccaa gctggagctg aaacgggctg atgctgcacc aactgtatcc      420
   atcttcccac catccagtga gcagttaaca tctggaggtg cctcagtcgt gtgcttcttg      480
   aacaacttct accccaaaga catcaatgtc aagtggaaga ttgatggcag tgaacgacaa      540
   aatggcgtcc tgaacagttg gactgatcag gacagcaaag acagcaccta cagcatgagc      600
   agcaccctca cgttgaccaa ggacgagtat gaacgacata acagctatac ctgtgaggcc      660
   actcacaaga catctacttc acccattgtc aagagcttca acaggaatga gtgttag         717
<210> 20
   <211> 10
   <212> PRT
   <213> artificial
<220>
   <223> DN30 light chain - CDR-L1
<400> 20
   Gln Ser Val Asp Tyr Asp Gly Gly Ser Tyr
   1               5                   10
<210> 21
   <211> 3
   <212> PRT
   <213> artificial
<220>
   <223> DN30 light chain - CDR-L2
<400> 21
   Ala Ala Ser
   1
<210> 22
   <211> 9
   <212> PRT
   <213> artificial
<220>
   <223> DN30 light chain - CDR-L3
<400> 22
   Gln Gln Ser Tyr Glu Asp Pro Leu Thr
   1               5
<210> 23
   <211> 31
   <212> DNA
   <213> artificial
<220>
   <223> CDR-L1 nucleotide
<400> 23
   aaagtgttga ttatgatggt ggtagttata t 31
<210> 24
   <211> 9
   <212> DNA
   <213> artificial
<220>
   <223> CDR-L2 nucleotide
<400> 24
   gctgcatcc 9
<210> 25
   <211> 27
   <212> DNA
   <213> artificial
<220>
   <223> CDR-L3 nucleotide
<400> 25
   cagcaaagtt atgaggatcc gctcacg 27
<210> 26
   <211> 21
   <212> DNA
   <213> artificial
<220>
   <223> primer NFKBIA
<400> 26
   gaaccccagc tcagggttta g 21
<210> 27
   <211> 20
   <212> DNA
   <213> artificial
<220>
   <223> primer rev. NFKBIA
<400> 27
   gggaatttcc aagccagtca 20
<210> 28
   <211> 21
   <212> DNA
   <213> artificial
<220>
   <223> primer kB1
<400> 28
   aggcccagtg ccttattacc a 21
<210> 29
   <211> 19
   <212> DNA
   <213> artificial
<220>
   <223> primer kB1 rev.
<400> 29
   gcggcctgac tggagattt 19
<210> 30
   <211> 24
   <212> DNA
   <213> artificial
<220>
   <223> primer kB2
<400> 30
   gggactcagt ttctttacct gcaa 24
<210> 31
   <211> 24
   <212> DNA
   <213> artificial
<220>
   <223> primer kB2 rev.
<400> 31
   gggactcagt ttctttacct gcaa 24
<210> 32
   <211> 199
   <212> DNA
   <213> homo sapiens
<400> 32
   atggtgtgaa ggacacctga ctgggctgaa agctaagttc taactttgcc cctcttacta    60
   accagctatg tgactctcct gggaactttt agggactcag tttctttacc tgcaaaatgg   120
   ttcaatgcaa gactttagta acgtaatggg aactttcctt ttccataaaa ctggggaatc   180
   aagaggtaat ctcttttga                                                199
<210> 33
   <211> 111
   <212> DNA
   <213> mus musculus
<400> 33
   cgtacgggct gttttattca tctgcaaaat gattccgtgc aggcctccaa aactgtaata        60
   ggaactttcc ttttccatca aactgaggag tggtgaggta aaccgctctt g                111